(19) Europäisches Patentamt
European Patent Office
Office européen des brevets

(11) Veröffentlichungsnummer: **0 274 353**
**B1**

(12) **EUROPÄISCHE PATENTSCHRIFT**

(45) Veröffentlichungstag der Patentschrift: **07.02.90**

(51) Int. Cl.⁴: **C07B 45/02,** C07C 303/28

(21) Anmeldenummer: **87810734.1**

(22) Anmeldetag: **09.12.87**

(54) **Verfahren zur Herstellung von Benzoinsulfonaten.**

(30) Priorität: **15.12.86 CH 4988/86**

(43) Veröffentlichungstag der Anmeldung:
**13.07.88 Patentblatt 88/28**

(45) Bekanntmachung des Hinweises auf die Patenterteilung:
**07.02.90 Patentblatt 90/6**

(84) Benannte Vertragsstaaten:
**DE ES FR GB IT**

(56) Entgegenhaltungen:
**DE-A- 1 919 678**

**BERICHTE DER DEUTSCHEN CHEMISCHEN GESELLSCHAFT, Band 60, 1927, Seiten 656-664, Berlin, DE; Z. FÖLDI: "Über die thermische Zersetzung der Sulfonsäure-ester"**

(73) Patentinhaber: **CIBA-GEIGY AG, Klybeckstrasse 141, CH-4002 Basel(CH)**

(72) Erfinder: **Rutsch, Werner, Dr., Avenue Weck-Reynold 1, CH-1700 Fribourg(CH)**
Erfinder: **Hüsler, Rinaldo, Dr., Route du Confin 52, CH-1723 Marly(CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

## Beschreibung

Die Erfindung betrifft ein Verfahren zur Herstellung von Benzoinestern aromatischer Sulfonsäuren, insbesondere von kristallinen Benzoinsulfonaten.

Solche Benzoinsulfonate sind aus der EP-A-84515 und EP-A-132 225 bekannt und werden dort als latente Härtungskatalysatoren für säurehärtbare Harze vorgeschlagen, die thermisch oder photolytisch aktiviert werden können. Für diese Verwendung ist es wichtig, dass die Ester frei von Sulfonsäure sind. Sie können auch als Ausgangsmaterialien für die Herstellung von 4,5-Diphenylthiazolinen und -oxazolinen verwendet werden, welche Zwischenprodukte für die Herstellung gewisser Cyanifarbstoffe sind.

Die Herstellung von Sulfonsäureestern erfolgt üblicherweise aus den entsprechenden Alkoholen und Sulfonsäurechloriden in Gegenwart eines HCl-Akzeptors. Dieses allgemeine Verfahren hat man auch zur Herstellung von Sulfonaten des Benzoins und seiner Derivate angewandt.

So beschreibt Z. Földi in Ber. 60, 664 (1927) die Umsetzung von Benzoin mit Benzolsulfochlorid in Benzol unter Zusatz von festem NaOH. Nach Waschen mit Wasser und Eindampfen der Benzolphase wird das rohe Sulfonat in 36 % Ausbeute erhalten.

I.J. Borowitz et al. beschreiben im Journal Org. Chem. 34, 1599 (1969) die Umsetzung von Benzoin mit Methansulfochlorid in Benzol in Gegenwart von 2 Aequivalenten Triethylamin. Nach Abtrennen des Aminsalzes wird die Benzolphase mit Wasser gewaschen und eingedampft. Nach Umkristallisation aus Ethylacetat wird das Benzoinmesylat in 69 % Ausbeute gewonnen. Wenn man in analoger Weise Benzoin in Benzol mit Toluolsulfochlorid und 2 Aequivalenten Triethylamin umsetzt, so verläuft die Reaktion bei Raumtemperatur sehr langsam. Nach 20 Stunden sind noch etwa 50 % des Benzoins vorhanden. Beschleunigt man die Reaktion durch Erwärmen, so treten Nebenreaktionen ein und unter anderen wird Benzil als Nebenprodukt gebildet. Insgesamt vermögen also die bisher bekannten Verfahrensweisen für die Herstellung von Benzoinestern von aromatischen Sulfonsäuren nicht zu befriedigen.

Da sowohl die verwendeten Sulfochloride wie auch die gebildeten Sulfonsäureester hydrolyseempfindlich sind, wird die Umsetzung von Sulfochloriden mit Benzoinen allgemein möglichst wasserfrei durchgeführt. Es wurde überraschend gefunden, dass man die Reaktion unter bestimmten Bedingungen auch in Gegenwart von Wasser durchführen kann und dass sich unter diesen Bedingungen die Gegenwart von Wasser im Sinne einer Reaktionsbeschleunigung und einer Ausbeuteerhöhung auswirkt. Die Bedingungen hierfür sind, dass während der Reaktion nie ein grösserer Basenüberschuss vorhanden ist und dass man die Reaktion bei relativ niedrigen Temperaturen ausführt. Die Reaktion wird in einem inerten Lösungsmittel ausgeführt.

Gegenstand der Erfindung ist ein Verfahren zur Herstellung von Sulfonsäureestern der Formel I,

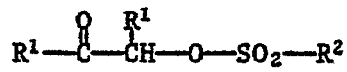

(I)

worin $R^1$ unsubstituiertes oder durch Halogen, $C_1$-$C_4$-Alkyl, $C_1$-$C_4$-Alkoxy, $C_1$-$C_4$-Alkylthio, -NH-CO-($C_1$-$C_4$-Alkyl), -NH-CO-Phenyl, Morpholino, Piperidino oder einen Rest -N($R^3$)($R^4$) substituiertes Phenyl oder unsubstituiertes oder durch Halogen oder $C_1$-$C_6$-Alkyl substituiertes Naphthyl oder Tetrahydronaphthyl oder einen unsub stituierten oder durch Halogen oder $C_1$-$C_4$-Alkyl substituierten einwertigen O-, S-oder N-haltigen 5- oder 6-gliedrigen heteroaromatischen Rest bedeutet,
$R^2$ unsubstituiertes oder durch Halogen, $C_1$-$C_{20}$-Alkyl, $C_1$-$C_4$-Halogenalkyl, $C_1$-$C_4$-Alkoxy, $C_1$-$C_4$-Alkylthio, Nitro, -NH-CO-($C_1$-$C_4$-Alkyl), -NH-CO-Phenyl oder Benzoyl substituiertes Phenyl oder unsubstituiertes oder durch Halogen oder $C_1$-$C_{20}$-Alkyl substituiertes Naphthyl oder einen unsubstituierten oder durch Halogen oder $C_1$-$C_6$-Alkyl substituierten O-, S- oder N-haltigen 5-oder 6-gliedrigen einwertigen heteroaromatischen Rest bedeutet, und
$R^3$ und $R^4$ unabhängig voneinander $C_1$-$C_4$-Alkyl, Allyl oder Cyclohexyl bedeuten,
durch Reaktion einer Benzoinverbindung der Formel II

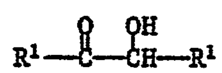

(II)

mit einem Sulfonsäurehalogenid der Formel III,

$$R^2—SO_2X \quad (III)$$

worin X Fluor, Chlor oder Brom bedeutet, in einem inerten organischen Lösungsmittel in Gegenwart einer Base, dadurch gekennzeichnet, dass man die Reaktion in Gegenwart von Wasser ausführt, dass man pro Mol III 1 bis 1,5 Aequivalente einer Base verwendet und dass man die Reaktion unterhalb 40°C ausführt.

In Formel I und II ist $R^1$ ein gegebenenfalls substituierter carbocyclischer oder heterocyclisher aromatischer Rest und kann z.B. Phenyl, Fluorphenyl, Chlorphenyl, Bromphenyl, Dichlorphenyl, Tolyl, Xylyl, Ethylphenyl, Isopropylphenyl, tert.Butylphenyl, Methoxyphenyl, Ethoxyphenyl, Butoxyphenyl, Dimethoxyphenyl, Methylthiophenyl, Butylthiophenyl, Acetylaminophenyl, Propionylaminophenyl, Benzoylaminophenyl, Dimethylaminophenyl, Dibutylaminophenyl, Methyl-cyclohexylaminophenyl, Naphthyl, Chlornaphthyl, Bromnaphthyl, Methylphthyl, Isopropylnaphthyl, Hexylnaphthyl, Tetrahydronaphthyl, Ethyl-tetrahydronaphthyl, Furyl, Thienyl, Pyridyl oder Methylpyridyl sein.

Bevorzugt ist $R^1$ unsubstituiertes oder durch Halogen, $C_1$-$C_4$-Alkyl, $C_1$-$C_4$-Alkoxy, oder $C_1$-$C_4$-Alkylthio substituiertes Phenyl, unsubstituiertes oder durch $C_1$-$C_6$-Alkyl substituiertes Naphthyl, Furyl, Pyridyl oder Thienyl, insbesondere Phenyl, Chlorphenyl oder Tolyl.

In Formel I und III ist $R^2$ ein gegebenenfalls substituierter carbocyclischer oder heterocyclisher aromatischer Rest und kann z.B. Phenyl, Chlorphenyl, Bromphenyl, Chlormethyl-phenyl, Trifluormethyl-phenyl, Tolyl, Xylyl, tert.Butylphenyl, Hexylphenyl, Octylphenyl, Nonylphenyl, Dodecylphenyl, Trimethylphenyl, Hexadecylphenyl, Octadecylphenyl, Methoxyphenyl, Ethoxyphenyl, Butoxyphenyl, Methylthiophenyl, Ethylthiophenyl, tert.Butylthiophenyl, Nitrophenyl, Acetylaminophenyl, Butyrylaminophenyl, Benzoylaminophenyl, Benzoylphenyl, Naphthyl, Chlornaphthyl, Bromnaphthyl, Methylnaphthyl, Butylnaphthyl, Hexylnaphthyl, Octylnaphthyl, Nonylnaphthyl, Decylnaphthyl, Dodecylnaphthyl, Octadecylnaphthyl, Eikosylnaphthyl, Furyl, Thienyl oder Pyridyl sein.

Bevorzugt ist $R^2$ unsubstituiertes oder durch Halogen, $C_1$-$C_{14}$-Alkyl, Halogenmethyl, $C_1$-$C_4$-Alkoxy, Nitro oder -NH-CO-($C_1$-$C_4$-Alkyl) substituiertes Phenyl, Naphthyl oder Pyridyl, insbesondere Phenyl, Bromphenyl, Chlorphenyl, Tolyl oder Naphthyl.

Bevorzugt verwendet man als Sulfonsäurehalogenid ein Sulfonsäurechlorid der Formel III, worin X Chlor ist.

Beispiele für erfindungsgemäss herstellbare Sulfonsäureester sind die folgenden Verbindungen:

2-[(Tolylsulfonyl)oxy]-1,2-diphenyl-1-ethanon
2-[(Phenylsulfonyl)oxy]-1,2-diphenyl-1-ethanon
2-[(β-Naphthylsulfonyl)oxy]-1,2-diphenyl-1-ethanon
2-[(m-Nitrophenylsulfonyl)oxy]-1,2-diphenyl-1-ethanon
2-[(2,4,6-Trimethylphenylsulfonyl)oxy]-1,2-diphenyl-1-ethanon
2-[(p-Bromphenylsulfonyl)oxy]-1,2-diphenyl-1-ethanon
2-[(Tolylsulfonyl)oxy]-1,2-bis-(o-chlorphenyl)-1-ethanon
2-[(Tolylsulfonyl)oxy]-1,2-bis-(p-methylphenyl)-1-ethanon

Die Umsetzung von II mit III erfolgt in annähernd äquimolarem Verhältnis, das Sulfonsäurehalogenid kann in einem leichten Ueberschuss verwendet werden. Vorzugsweise arbeitet man mit einem Molverhältnis II:III von 1:1 bis 1:1,3.

Als Base kann z.B. ein Hydroxid, Oxid oder Alkoholat eines Alkali- oder Erdalkalimetalles verwendet werden. Vorzugsweise verwendet man eine wasserlösliche Base, insbesondere wässrige NaOH oder KOH. Die Base wird dem Reaktionsgemisch so zugegeben, dass nie ein grösserer Ueberschuss an Base vorliegt. Vorzugsweise verwendet man pro Mol III 1 bis 1,2 Aequivalente Base.

Als Lösungsmittel kann ein polares oder unpolares Lösungsmittel verwendet werden. Polare Lösungsmittel sind meist mit Wasser mischbar, so dass die Reaktion einphasig verlaufen kann. Unpolare Lösungsmittel sind mit Wasser meist nicht mischbar, so dass sich im Laufe der Reaktion zwei flüssige Phasen bilden können.

In jedem Fall müssen die Lösungsmittel inert gegenüber Sulfosäurehalogeniden sein, sie dürfen also z.B. keine nucleophilen OH- oder NH-Gruppen besitzen. Beispiele für brauchbare Lösungsmittel sind Benzol, Toluol, Xylol, Chlorbenzol, Nitrobenzol, Dichlorbenzol, Methylenchlorid, Tetrachlorethan, Dibutylether, Methyl-ethyl-keton, Methylisobutyl-keton, Aceton, Tetrahydrofuran, Dioxan, Acetonitril, Dimethylformamid, Dimethylacetamid oder N-Methylpyrrolidon, bevorzugt verwendet man Methyl-ethyl-keton, Toluol, Xylol, Methylenchlorid, Chlorbenzol, Dichlorbenzol, Dibutylether, Methyl-isobutylketon, Aceton, Dioxan, Tetrahydrofuran oder Dimethylformamid.

Besonders geeignet sind Lösungsmittel mittlerer Polarität, die eine gewisse Menge Wasser aufnehmen können, wie z.B. Methyl-ethyl-keton, Aceton oder Tetrahydrofuran.

Die Reaktion kann durch Katalysatoren beschleunigt werden, wie sie für die Umsetzung von Säurehalogeniden mit Alkoholen bekannt sind, beispielsweise mit p-Dimethylaminopyridin.

Wenn die Reaktion zweiphasig ausgeführt wird, so ist es von Vorteil, einen Phasentransfer-Katalysator zuzusetzen, wie z.B. ein quaternäres Ammonium- oder Phosphoniumsalz, einen Polyalkylenether oder einen Kronenether.

Da die meisten Benzoinsulfonate kristalline Verbindungen sind, kann ihre Isolation aus dem Reaktionsgemisch im einfachsten Fall durch direkte Kristallisation geschehen. Oft beginnt das Sulfonat gegen Ende der Reaktion von selbst aus der Reaktionslösung auszukristallisieren.

Die Kristallisation kann durch Kühlen des Reaktionsgemisches beschleunigt werden. Die jeweilige Menge des Lösungsmittels und des Wasserzusatzes soll so gewählt werden, dass am Ende der Reaktion das Basensalz vollständig gelöst bleibt, während das Benzoinsulfonat möglichst vollständig auskristallisieren soll.

Das Verhältnis von Wasser:Lösungsmitteln beträgt nach der Zugabe der Base 1:100 bis 10:1, bevorzugt 1:25 bis 1:1.

Ist das Benzoinsulfonat flüssig, so arbeitet man zweckmässig in einem mit Wasser nicht mischbaren Lösungsmittel. Nach der Reaktion trennt man die wässrige Schicht ab und isoliert das Benzoinsulfonat durch Eindampfen der organischen Phase. Vor dem Eindampfen sollte die organische Phase neutral gewaschen und getrocknet werden. Auch sollte das Eindampfen bei möglichst niedriger Temperatur geschehen um eine Hydrolyse des Benzoinsulfonates zu vermeiden.

Unabhängig vom verwendeten Lösungsmittel und der Base soll die Reaktion bei relativ niedriger Temperatur ausgeführt werden. Bevorzugt arbeitet man bei 0° bis +25°C, was eine Kühlung des Reaktionsmediums erfordert.

Ein wesentlicher Vorteil des erfindungsgemässen Verfahrens in Gegenwart von Wasser gegenüber einer wasserfreien Verfahrensweise ist, dass keine Abtrennung fester Basen oder Basensalze notwendig ist und daher der Sulfonsäureester direkt aus der Reaktionslösung durch Kristallisation isoliert werden kann. Ein weiterer Vorteil ist, dass das verwendete Lösungsmittel nicht wasserfrei zu sein braucht, es entfällt also eine spezielle Trocknung des Lösungsmittels und des Reaktionsgefässes vor der Reaktion. Das Verfahren liefert die gewünschten Sulfonsäureester in hoher Ausbeute und hoher Reinheit.

Die nachstehenden Beispiele geben weitere Details des Verfahrens, ohne es auf die Verfahrensweise der Beispiele einzuschränken.

Beispiel 1 - Benzoin-p-toluolsulfonat

In einem 2,5 l Sulfierkolben werden 650 ml Methylethylketon unter Rühren mit 424,5 g (2,0 Mol) Benzoin versetzt. Die entstandene beige Suspension wird 5 Minuten lang gerührt, und anschliessend werden 400,4 g (2,1 Mol) pulverisiertes p-Toluolsulfonsäurechlorid zugegeben. Es findet noch keine chemische Reaktion statt. Die Temperatur sinkt von Raumtemperatur auf ca. +5°C. Nach beendeter Zugabe wird die Suspension während 5 Minuten weitergerührt und anschliessend mit 50 ml deionisiertem Wasser versetzt. Innerhalb von 30 Minuten werden nun 293,3 g 30%ige wässrige Natronlauge (entsprechend 2,2 Mol NaOH) zugetropft, wobei die Temperatur durch Kühlung mit einem Eisbad bei 10-15°C gehalten wird. Die beige Suspension wird bei dieser Temperatur 6 Stunden intensiv gerührt, dann auf -10° bis -15°C abgekühlt und 1 Stunde bei dieser Temperatur kräftig weitergerührt. Der bis zum Schluss gut rührbare beige Kristallbrei wird abfiltriert, 1 bis 3 mal mit je 200 ml deionisiertem Wasser und anschliessend einmal mit 150 ml wasserhaltigem eiskaltem Methylethylketon gewaschen. Nach Trocknen im Vakuum bei Raumtemperatur erhält man 674 g (92 % der Theorie) Benzoin-p-toluolsulfonat in Form weisser Kristalle, die bei 99-101°C schmelzen. Gemäss Dünnschicht-Chromatographie und NMR-Spektrum beträgt die Reinheit mindestens 98 %. Eine Probe in Wasser angerührt zeigt mit pH-Papier keine saure Reaktion.

Analyse $C_{21}H_{18}O_4S$

berechnet: 68,83 % C 4,95 % H 8,75 % S

gefunden : 68,61 % C 4,98 % H 8,63 % S

In analoger Weise lässt sich die Reaktion in wässrigem Aceton ausführen. Hierbei werden 0,1 Mol Benzoin in 30 ml Aceton + 5 ml Wasser mit 0,105 Mol p-Toluolsulfochlorid un 0,11 Mol 30%iger wässriger NaOH 7 Stunden bei 10-15°C umgesetzt und man erhält durch direkte Kristallisation aus der Reaktionslösung das Sulfonat in 91 % Ausbeute.

Beispiel 2 - Benzoin-p-brombenzolsulfonat

Analog zu Beispiel 1 werden 0,2 Mol Benzoin in 65 ml Methylethylketon unter Zusatz von 5 ml Wasser mit 0,22 Mol p-Brombenzolsulfochlorid und 0,24 Mol 30%iger wässriger NaOH bei 10-15°C umgesetzt. Durch Filtration des Reaktionsgemisches und Waschen mit Wasser und kaltem Methyl-ethyl-keton erhält man nach Trocknen im Vakuum das gewünschte Sulfonat mit einem Smp. von 114-122°C.

Bei analoger Umsetzung von 0,1 Mol Benzoin mit 0,105 Mol p-Brombenzolsulfochlorid in 30 ml Tetrahydrofuran und 5 ml Wasser unter Zusatz von 0,11 Mol 30%iger NaOH erhält man dieselbe Verbindung.

Beispiel 3 - Benzoin-m-nitrobenzolsulfonat

In Analogie zu Beispiel 2 wird 3-Nitrobenzolsulfochlorid mit Benzoin in Methylethylketon und wässriger NaOH umgesetzt. Das erhaltene Sulfonat schmilzt bei 92-96°C.

Beispiel 4- 4,4'-Dichlorbenzoin-toluolsulfonat

Analog Beispiel 2 wird p-Toluolsulfochlorid mit 4,4'-Dichlorbenzoin in 200 ml Methylethylketon umgesetzt. Das erhaltene Sulfonat schmilzt bei 129-130°C.

Beispiel 5 - 4,4'-Dimethylbenzoin-toluolsulfonat

Analog Beispiel 4 erhält man das Sulfonat, das bei 94-95°C schmilzt.

Beispiel 6 - Benzoin-β-naphthylsulfonat

In 500 ml Tetrahydrofuran werden 0,1 Mol Benzoin und 0,12 Mol β-Naphthalinsulfochlorid bei Raumtemperatur gelöst. Nach Zusatz von 0,15 Mol 30%iger Natronlauge wird 8 h bei Raumtemperatur gerührt. Die Reaktionslösung wird mit 200 ml Methylenchlorid verdünnt und die organische Phase je einmal mit $NaHCO_3$-Lösung und Wasser gewaschen. Die organische Lösung wird über $MgSO_4$ getrocknet und eingedampft. Der Rückstand wird aus Hexan/Ethylacetat umkristallisiert. Das erhaltene Sulfonat schmilzt bei 107-109°C.

Beispiel 7 - Benzoin-benzolsulfonat

Analog Beispiel 6 erhält man das Sulfonat aus Benzoin und Benzolsulfochlorid. Smp. 89-90°C.

**Patentansprüche**

1. Verfahren zur Herstellung von Sulfonsäureestern der Formel I,

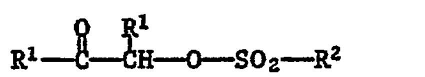

(I)

worin $R^1$ unsubstituiertes oder durch Halogen, $C_1$-$C_4$-Alkyl, $C_1$-$C_4$-Alkoxy, $C_1$-$C_4$-Alkylthio, -NH-CO-($C_1$-$C_4$-Alkyl), -NH-CO-Phenyl, Morpholino, Piperidino oder einen Rest -N($R^3$)($R^4$) substituiertes Phenyl oder unsubstituiertes oder durch Halogen oder $C_1$-$C_6$-Alkyl substituiertes Naphthyl oder Tetrahydronaphthyl oder einen unsubstituierten oder durch Halogen oder $C_1$-$C_4$-Alkyl substituierten einwertigen O-, S-oder N-haltigen 5- oder 6-gliedrigen heteroaromatischen Rest bedeutet,

$R^2$ unsubstituiertes oder durch Halogen, $C_1$-$C_{20}$-Alkyl, $C_1$-$C_4$-Halogenalkyl, $C_1$-$C_4$-Alkoxy, $C_1$-$C_4$-Alkylthio, Nitro, -NH-CO-($C_1$-$C_4$-Alkyl), -NH-CO-Phenyl oder Benzoyl substituiertes Phenyl oder unsubstituiertes oder durch Halogen oder $C_1$-$C_{20}$-Alkyl substituiertes Naphthyl oder einen unsubstituierten oder durch Halogen oder $C_1$-$C_6$-Alkyl substituierten O-, S- oder N-haltigen 5-oder 6-gliedrigen

einwertigen heteroaromatischen Rest bedeutet, und $R^3$ und $R^4$ unabhängig voneinander $C_1$-$C_4$-Alkyl, Allyl oder Cyclohexyl bedeuten,
durch Reaktion einer Benzoinverbindung der Formel II

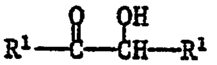 (II)

mit einem Sulfonsäurehalogenid der Formel III,

$R^2$—$SO_2X$ (III)

worin X Fluor, Chlor oder Brom bedeutet, in einem inerten organischen Lösungsmittel in Gegenwart einer Base, dadurch gekennzeichnet, dass man die Reaktion in Gegenwart von Wasser ausführt, dass man pro Mol III bis 1,5 Aequivalente einer Base verwendet und dass man die Reaktion unterhalb 40°C ausführt.

2. Verfahren gemäss Anspruch 1, dadurch gekennzeichnet, dass man eine Verbindung der Formel II verwendet, worin $R^1$ unsubstituiertes oder durch Halogen, $C_1$-$C_4$-Alkyl, $C_1$-$C_4$-Alkoxy oder $C_1$-$C_4$-Alkylthio substituiertes Phenyl, unsubstituiertes oder durch $C_1$-$C_6$-Alkyl substituiertes Naphthyl, Furyl, Pyridyl oder Thienyl bedeutet.

3. Verfahren gemäss Anspruch 1, dadurch gekennzeichnet, dass man eine Verbindung der Formel II verwendet, worin $R^1$ Phenyl, Chlorphenyl oder Tolyl ist.

4. Verfahren gemäss Anspruch 1, dadurch gekennzeichnet, dass man eine Verbindung der Formel III verwendet, worin X Chlor ist.

5. Verfahren gemäss Anspruch 1, dadurch gekennzeichnet, dass man eine Verbindung der Formel III verwendet, worin $R^2$ unsubstituiertes oder durch Halogen, $C_1$-$C_{20}$-Alkyl, Halogenmethyl, $C_1$-$C_4$-Alkoxy, Nitro oder -NH-CO-($C_1$-$C_4$-Alkyl) substituiertes Phenyl, Naphthyl oder Pyridyl bedeutet.

6. Verfahren gemäss Anspruch 1, dadurch gekennzeichnet, dass man eine Verbindung der Formel III verwendet, worin $R^2$ Phenyl, Bromphenyl, Nitrophenyl, Tolyl oder Naphthyl bedeutet.

7. Verfahren gemäss Anspruch 1, dadurch gekennzeichnet, dass man die Reaktion bei 0°C bis +25°C ausführt.

8. Verfahren gemäss Anspruch 1, dadurch gekennzeichnet, dass man als Base eine wässrige Lösung von NaOH oder KOH verwendet.

9. Verfahren gemäss Anspruch 1, dadurch gekennzeichnet, dass man als Lösungsmittel Methylethylketon, Toluol, Xylol, Methylenchlorid, Chlorbenzol, Dichlorbenzol, Dibutylether, Methylisobutylketon, Aceton, Acetonitril, Dioxan, Tetrahydrofuran oder Dimethylformamid verwendet.

10. Verfahren gemäss Anspruch 1, dadurch gekennzeichnet, dass man als Lösungsmittel Methylethylketon, Aceton oder Tetrahydrofuran verwendet.

## Claims

1. A process for the preparation of a sulfonic acid ester of formula I

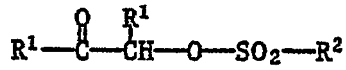 (I)

in which
$R^1$ is phenyl which is unsubstituted or substituted by halogen, $C_1$–$C_4$alkyl, $C_1$–$C_4$alkoxy, $C_1$–$C_4$alkylthio, –NH–CO–($C_1$–$C_4$alkyl), –NH-CO-phenyl, morpholino, piperidino or a radical –N($R^3$) ($R^4$), or is naphthyl or tetrahydronaphthyl, each unsubsituted or substituted by halogen or $C_1$–$C_6$alkyl, or is a univalent 5- or 6- membered heteroaromatic radical which contains O, S or N and is unsubstituted or substituted by halogen or $C_1$–$C_4$alkyl,
$R^2$ is phenyl which is unsubstituted or substituted by halogen, $C_1$–$C_{20}$alkyl, $C_1$–$C_4$haloalkyl, $C_1$–$C_4$alkoxy, $C_1$–$C_4$alkylthio, nitro, –NH–CO–($C_1$–$C_4$alkyl), –NH–CO-phenyl or benzoyl, or is naphthyl which is unsubstituted or substituted by halogen or $C_1$–$C_{20}$alkyl, or is a univalent 5- or 6- membered heteroaromatic radical which contains O, S or N and is unsubstituted or substituted by halogen or $C_1$–$C_6$alkyl, and
$R^3$ and $R^4$ are each independenty of the other $C_1$–$C_4$alkyl, allyl or cyclohexyl,
by reaction of a benzoin compound of formula II

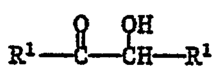 (II)

with a sulfonyl halide of formula III

$R^2$—$SO_2X$ (III)

in which X is flourine, chlorine or bromine, in an inert organic solvent and in the presence of a base, which process comprises carrying out the reaction in the presence of water, using 1 to 1.5 equivalents of a base per mole of III, and carrying out the reaction at below 40°C.

2. A process according to claim 1, which comprises the use of a compound of formula II, in which $R^1$ is phenyl which is unsubstituted or substituted by halogen, $C_1$–$C_4$alkoxy or $C_1$–$C_4$alkythio, or naphthyl, furyl, pyridyl or thienyl, each unsubstituted or substituted by $C_1$–$C_6$alkyl.

3. A process according to claim 1, which comprises the use of a compound of formula II, in which $R^1$ is phenyl, chlorophenyl or tolyl.

4. A process according to claim 1, which comprises the use of a compound of formula III, in which X is chlorine.

5. A process according to claim 1, which comprises the use of a compound of formula III, in which $R^2$ is phenyl, naphthyl or pyridyl, each unsubstituted or substituted by halogen, $C_1$–$C_{20}$alkyl, halomethyl, $C_1$–$C_4$alkoxy, nitro or –NH–CO–($C_1$–$C_4$alkyl).

6. A process according to claim 1, which comprises the use of a compound of formula III, in which $R^2$

is phenyl, bromophenyl, nitrophenyl, tolyl or naphthyl.

7. A process according to claim 1, wherein the reaction is carried out at 0°C to +25°C.

8. A process according to claim 1, wherein the base used is an aqueous solution of NaOH or KOH.

9. A process according to claim 1, wherein the solvent used is methyl ethyl ketone, toluene, xylene, methylene chloride, chlorobenzene, dichlorobenzene, dibutyl ether, methyl isobutyl ketone, acetone, acetonitrile, dioxane, tetrahydrofuran or dimethylformamide.

10. A process according to claim 1, wherein the solvent used is methyl ethyl ketone, acetone or tetrahydrofuran.

**Revendications**

1. Procédé pour préparer des esters d'acides sulfoniques répondant à la formule I

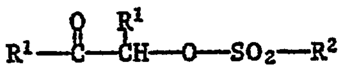 (I)

dans laquelle
$R^1$ représente un radical phényle non substitué ou porteur d'un halogène, d'un alkyle en $C_1$–$C_4$, d'un alcoxy en $C_1$–$C_4$, d'un alkylthio en $C_1$–$C_4$, d'un –NH–CO–($C_1$–$C_4$-alkyl), d'un –NH–CO-phényl, d'un morpholino, d'un pipéridino ou d'un radical –N($R^3$) ($R^4$), ou un radical naphtyle ou tétrahydronaphtyle non substitué ou porteur d'un halogène ou d'un alkyle en $C_1$–$C_6$, ou un radical hétéro-aromatique univalent à 5 ou 6 maillons qui contient O, S ou N, ce radical étant dépourvu de substituant ou portant un halogène ou un alkyle en $C_1$–$C_4$,
$R^2$ représente un radical phényle non substitué ou porteur d'un halogène, d'un alkyle en $C_1$ à $C_{20}$, d'un halogèno-alkyle en $C_1$–$C_4$, d'un alcoxy en $C_1$–$C_4$, d'un alkylthio en $C_1$–$C_4$, d'un nitro, d'un –NH–CO–($C_1$–$C_4$-alkyl), d'un –NH–CO-phényl ou d'un benzoyle, un radical naphtyle non substitué ou porteur d'un halogène ou d'un alkyle en $C_1$ à $C_{20}$, ou un radical hétéro-aromatique univalent à 5 ou 6 maillons contenant O, S ou N, ce radical étant dépourvu de substituant ou portant un halogène ou un alkyle en $C_1$ à $C_6$, et
$R^3$ et $R^4$ représentent chacun, indépendamment l'un de l'autre, un alkyle en $C_1$ à $C_4$, un allyle ou un oyclohexyle,
par réaction d'une benzoïne de formule II

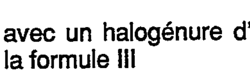 (II)

avec un halogénure d'acide sulfonique répondant à la formule III

$$R^2\text{—}SO_2X \quad \text{(III)}$$

dans laquelle X représente le flour, le chlore ou le brome, dans un solvant organique inerte, en présence d'une base, procédé caractérisé en ce qu'on effectue la réaction en présence d'eau, en ce qu'on utilise de 1 à 1,5 équivalent d'une base par mole du composé (III), et en ce qu'on effectue la réaction à une température inférieure à 40°C.

2. Procédé selon la revendication 1 caractérisé en ce qu'on utilise un composé de formule II dans lequel $R^1$ repésente un radical phényle non substitué ou porteur d'un halogène, d'un alkyle en $C_1$–$C_4$, d'un alcoxy en $C_1$–$C_4$ ou d'un alkylthio en $C_1$–$C_4$, un radical naphtyle non substitué ou porteur d'un alkyle en $C_1$ à $C_6$, ou un radical furyle, pyridyle ou thiényle.

3. Procédé selon la revendication 1 caractérisé en ce qu'on utilise un composé de formule II dans lequel $R^1$ représente une phényle, un chlorophényle ou un tolyle.

4. Procédé selon la revendication 1 caractérisé en ce qu'on utilise un composé de formule III dans lequel X représente le chlore.

5. Procédé selon la revendication 1 caractérisé en ce qu'on utilise un composé de formule III dans lequel $R^2$ représente un radical phényle non substitué ou porteur d'un halogène, d'un alkyle en $C_1$ à $C_{20}$, d'un halogénométhyle, d'un alcoxy en $C_1$ à $C_4$, d'un nitro ou d'un –NH–CO-(alkyl en $C_1$–$C_4$), un radical naphtyle ou un radical pyridyle.

6. Procédé selon la revendication 1 caractérisé en ce qu'on utilise un composé de formule III dans lequel $R^2$ représente un radical phényle, bromophényle, nitrophényle, tolyle ou naphtyle.

7. Procédé selon la revendication 1 caractérisé en ce qu'on effectue la réaction à une température de 0 à +25° C.

8. Procédé selon la revendication 1 caractérisé en ce qu'on utilise, comme base, une solution aqueuse de NaOH ou de KOH.

9. Procédé selon la revendication 1 caractérisé en ce qu'on utilise, comme solvant, la méthyl-éthylcétone, le toluène, un xylène, le chlorure de méthylène, le chlorobenzène, un dichlorobenzène, l'oxyde de dibutyle, la méthyl-isobutyl-cétone, l'acétone, l'acétonitrile, le dioxanne, le tétrahydrofuranne ou le diméthylformamide.

10. Procédé selon la revendication 1 caractérisé en ce qu'on utilise, comme solvant, la méthyl-éthylcétone, l'acétone ou le tétrahydrofuranne.